(19) **Europäisches Patentamt European Patent Office Office européen des brevets**

(11) **EP 4 181 901 B1**

(12) # EUROPEAN PATENT SPECIFICATION

(45) Date of publication and mention
of the grant of the patent:
**15.10.2025 Bulletin 2025/42**

(21) Application number: **21751757.2**

(22) Date of filing: **15.07.2021**

(51) International Patent Classification (IPC):
*A61K 31/05* (2006.01)    *A61K 31/197* (2006.01)
*A61K 31/423* (2006.01)    *A61K 31/515* (2006.01)
*A61K 31/551* (2006.01)    *A61K 31/5513* (2006.01)
*A61K 45/06* (2006.01)    *A61P 25/08* (2006.01)

(52) Cooperative Patent Classification (CPC):
(C-Sets available)
**A61K 45/06; A61K 31/05; A61K 31/197;
A61K 31/423; A61K 31/515; A61K 31/551;
A61K 31/5513; A61K 31/658; A61P 25/08** (Cont.)

(86) International application number:
**PCT/EP2021/069900**

(87) International publication number:
**WO 2022/017959 (27.01.2022 Gazette 2022/04)**

(54) **USE OF CANNABIDIOL IN THE TREATMENT OF SEIZURES ASSOCIATED WITH RARE EPILEPSY SYNDROMES RELATED TO GENETIC ABNORMALITIES**

VERWENDUNG VON CANNABIDIOL BEI DER BEHANDLUNG VON ANFÄLLEN IM ZUSAMMENHANG MIT SELTENEN EPILEPSIESYNDROMEN IM ZUSAMMENHANG MIT GENETISCHEN ANOMALIEN

UTILISATION DE CANNABIDIOL DANS LE TRAITEMENT DE CRISES ASSOCIÉES À DES SYNDROMES D'ÉPILEPSIES RARES ASSOCIÉS À DES ANOMALIES GÉNÉTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2020 GB 202011160**

(43) Date of publication of application:
**24.05.2023 Bulletin 2023/21**

(73) Proprietor: **Jazz Pharmaceuticals Research UK Limited
Sittingbourne, Kent ME9 8AG (GB)**

(72) Inventors:
 • **CHECKETTS, Daniel, Adam
 Sittingbourne, Kent ME9 8AG (GB)**
 • **CRAIG, Kevin, James
 Sittingbourne, Kent ME9 8AG (GB)**

(74) Representative: **HGF
HGF Limited
1 City Walk
Leeds LS11 9DX (GB)**

(56) References cited:
**WO-A1-2016/203239    WO-A1-2019/207319
GB-A- 2 531 282**

• **D'ADAMO MARIA CRISTINA ET AL: "Ion Channels Involvement in Neurodevelopmental Disorders", NEUROSCIENCE, NEW YORK, NY, US, vol. 440, 28 May 2020 (2020-05-28), pages 337 - 359, XP086210982, ISSN: 0306-4522, [retrieved on 20200528], DOI: 10.1016/ J.NEUROSCIENCE.2020.05.032**

**EP 4 181 901 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- BALESTRINI S ET AL: "Treatment of epileptic encephalopathies", vol. 23, no. 37, 1 October 2017 (2017-10-01), pages 5667 - 5690, XP009530524, ISSN: 1381-6128, Retrieved from the Internet <URL:http://www.eurekaselect.com/154786/article> DOI: 10.2174/1381612823666170809115827
- DEVINSKY ORRIN ET AL: "Cannabidiol in patients with treatment-resistant epilepsy: an open-label interventional trial", LANCET NEUROLOGY, LANCET PUBLISHING GROUP, LONDON, GB, vol. 15, no. 3, 24 December 2015 (2015-12-24), pages 270 - 278, XP029415431, ISSN: 1474-4422, DOI: 10.1016/S1474-4422(15)00379-8
- BORLOT FELIPPE ET AL: "KCNT1-related epilepsy: An international multicenter cohort of 27 pediatric cases", EPILEPSIA, vol. 61, no. 4, 13 March 2020 (2020-03-13), New York , US, pages 679 - 692, XP055832768, ISSN: 0013-9580, Retrieved from the Internet <URL:https://onlinelibrary.wiley.com/doi/full-xml/10.1111/epi.16480> DOI: 10.1111/epi.16480
- PAULHUS KELSEY ET AL: "Clinical Spectrum of KCNA1 Mutations: New Insights into Episodic Ataxia and Epilepsy Comorbidity", INTERNATIONAL JOURNAL OF MOLECULAR SCIENCES, vol. 21, no. 8, 17 April 2020 (2020-04-17), pages 2802, XP055855226, Retrieved from the Internet <URL:https://www.ncbi.nlm.nih.gov/pmc/articles/PMC7215408/pdf/ijms-21-02802.pdf> DOI: 10.3390/ijms21082802
- PISANO TIZIANA ET AL: "Early and effective treatment of KCNQ2 encephalopathy", EPILEPSIA, vol. 56, no. 5, 1 May 2015 (2015-05-01), New York , US, pages 685 - 691, XP055855385, ISSN: 0013-9580, DOI: 10.1111/epi.12984

(52) Cooperative Patent Classification (CPC): (Cont.)

C-Sets
A61K 31/05, A61K 2300/00;
A61K 31/197, A61K 2300/00;
A61K 31/423, A61K 2300/00;
A61K 31/515, A61K 2300/00;
A61K 31/551, A61K 2300/00;
A61K 31/5513, A61K 2300/00;
A61K 31/658, A61K 2300/00

**Description**

**FIELD OF THE INVENTION**

The present invention relates to a cannabidiol (CBD) preparation for use in the treatment of seizures associated with rare epilepsy syndromes. According to the claimed invention, the seizures associated with rare epilepsy syndromes that are treated are those which are experienced in patients with mutation in the potassium channel (KCN) genes KCNA1 and/or KCNQ2. In a further embodiment the types of seizures include tonic, tonic-clonic, absence and focal seizures with impairment. Preferably the dose of CBD is between 5 mg/kg/day to 50 mg/kg/day.

In a further embodiment the CBD used is in the form of a highly purified extract of cannabis such that the CBD is present at greater than 95% of the total extract (w/w) and the cannabinoid tetrahydrocannabinol (THC) has been substantially removed, to a level of not more than 0.15% (w/w).

Preferably the CBD used is in the form of a botanically derived purified CBD which comprises greater than or equal to 98% (w/w) CBD and less than or equal to 2% (w/w) of other cannabinoids. More preferably the other cannabinoids present are THC at a concentration of less than or equal to 0.1% (w/w); CBD-C1 at a concentration of less than or equal to 0.15% (w/w); CBDV at a concentration of less than or equal to 0.8% (w/w); and CBD-C4 at a concentration of less than or equal to 0.4% (w/w). The botanically derived purified CBD preferably also comprises a mixture of both trans-THC and cis-THC. Alternatively, a synthetically produced CBD is used.

Most preferably the other cannabinoids present are THC at a concentration of about 0.01% to about 0.1% (w/w); CBD-C1 at a concentration of about 0.1% to about 0.15% (w/w); CBDV at a concentration of about 0.2% to about 0.8% (w/w); and CBD-C4 at a concentration of about 0.3% to about 0.4% (w/w). Most preferably still the THC is present at a concentration of about 0.02% to about 0.05% (w/w).

Where the CBD is given concomitantly with one or more other anti-epileptic drugs (AED), the CBD may be formulated for administration separately, sequentially or simultaneously with one or more AED or the combination may be provided in a single dosage form.

**BACKGROUND TO THE INVENTION**

Epilepsy occurs in approximately 1% of the population worldwide, (Thurman *et al., 2011*) of which 70% are able to adequately control their symptoms with the available existing anti-epileptic drugs (AED). However, 30% of this patient group, (Eadie *et al., 2012*), are unable to obtain seizure freedom from the AED that are available and as such are termed as suffering from intractable or "treatment-resistant epilepsy" (TRE).

Intractable or treatment-resistant epilepsy was defined in 2009 by the International League Against Epilepsy (ILAE) as *"failure of adequate trials of two tolerated and appropriately chosen and used AED schedules (whether* as *monotherapies or in combination) to achieve sustained seizure freedom"* (Kwan *et al.,* 2009).

Individuals who develop epilepsy during the first few years of life are often difficult to treat and as such are often termed treatment resistant. Children who undergo frequent seizures in childhood are often left with neurological damage which can cause cognitive, behavioral and motor delays.

Childhood epilepsy is a relatively common neurological disorder in children and young adults with a prevalence of approximately 700 per 100,000. This is twice the number of epileptic adults per population.

When a child or young adult presents with a seizure, investigations are normally undertaken in order to investigate the cause. Childhood epilepsy can be caused by many different syndromes and genetic mutations and as such diagnosis for these children may take some time.

The main symptom of epilepsy is repeated seizures. In order to determine the type of epilepsy or the epileptic syndrome that a patient is suffering from an investigation into the type of seizures that the patient is experiencing is undertaken. Clinical observations and electroencephalography (EEG) tests are conducted and the type(s) of seizures are classified according to the ILEA classification.

Generalized seizures, where the seizure arises within and rapidly engages bilaterally distributed networks, can be split into six subtypes: tonic-clonic (grand mal) seizures; absence (petit mal) seizures; clonic seizures; tonic seizures; atonic seizures and myoclonic seizures.

Focal (partial) seizures where the seizure originates within networks limited to only one hemisphere, are also split into sub-categories. Here the seizure is characterized according to one or more features of the seizure, including aura, motor, autonomic and awareness / responsiveness. Where a seizure begins as a localized seizure and rapidly evolves to be distributed within bilateral networks this seizure is known as a bilateral convulsive seizure, which is the proposed terminology to replace secondary generalized seizures (generalized seizures that have evolved from focal seizures and are no longer remain localized).

Focal seizures where the subject's awareness / responsiveness is altered are referred to as focal seizures with impairment and focal seizures where the awareness or responsiveness of the subject is not impaired are referred to as focal seizures without impairment.

Potassium channels are the most widely distributed type of ion channel and are found in almost all living organisms, in most cell types including neurons, muscle cells, and other tissues, and control a wide variety of cell functions. They form potassium-selective pores that span cell membranes. There are four major classes of potassium channels: i) Calcium-activated potassium channel; ii) Inwardly rectifying potassium channel; iii) Tandem pore domain potassium channel; and iv) Voltage-gated potassium channel.

Voltage-gated potassium channels are transmembrane channels specific for potassium and sensitive to voltage changes in the cell's membrane potential. They play a fundamental role in the generation and propagation of the action potential.

Genes contained within the group of voltage-gated potassium channels include *KCNA1,* which encodes potassium voltage-gated channel subfamily A member 1, and KCNQ2, which encodes potassium voltage-gated channel subfamily Q member 2.

The *KCNA1* gene encodes the alpha subunit of a potassium channel called Kv1.1. These channels are found in the brain, where they transport potassium ions into neurons. The flow of certain ions, including potassium, into and out of neurons regulates communication between these cells.

Mutations in the *KCNA1* gene have been found in people with episodic ataxia type 1. People with this form of the condition have brief, recurrent episodes of poor coordination and balance. Between episodes, many affected individuals experience myokymia, a muscle abnormality that can cause involuntary muscle cramping, stiffness, and continuous, fine muscle twitching that appears as rippling under the skin. Changes in this gene have also been identified in a number of people with epilepsy. Treatment for episodic ataxia includes acetazolamide, carbamazepine and valproic acid.

The KCNQ2 gene encodes a protein present in channels in neurons in the brain. These channels transmit a particular type of electrical signal called the M-current, which prevents the neuron from continuing to send signals to other neurons.

Mutations in the KCNQ2 gene are associated with benign familial neonatal seizures, a condition characterized by recurrent seizures in newborn babies. The seizures begin around day 3 of life and usually go away within 1 to 4 months. Anticonvulsant therapy is needed to stop seizures in the neonatal period, particularly in cases with very frequent seizures or status epilepticus.

Mutations in the KCNQ2 gene have also been identified in early-onset epileptic encephalopathy, characterized by epilepsy and profound intellectual disability. The seizures begin in the first weeks of life and typically show little response to treatment. They usually go away in a few months to a few years but can return later in childhood. Most affected individuals are unable to talk, and they have low muscle tone or very stiff muscles, causing difficulty with movement.

Cannabidiol (CBD), a non-psychoactive derivative from the cannabis plant, has demonstrated anti-convulsant properties in several anecdotal reports, pre-clinical and clinical studies both in animal models and humans. Three randomized control trials showed efficacy of the purified pharmaceutical formulation of CBD in patients with Dravet and Lennox-Gastaut syndrome.

Several documents including GB2581517, Borlot *et al.* (2020)[1] and Poisson *et al.* (2020)[2] disclose the potential use of CBD in treating KCNT1-related epilepsy, specifically Epilepsy of Infancy with Migrating Focal Seizures (EIMFS). KCNT1 protein differs from KCNA1 and KCNQ2 proteins in that it is part of a class of sodium-activated potassium channels.

Documents such as WO 2019/045121, US 2019/091174 and US 2019/247333 mention use of compounds such as fenfluramine to treat seizures associated with mutations in KCNA1 and KCNQ2 genes. None discuss any use of cannabinoids let alone CBD. D'Adamo, et. al.: Neuroscience, 2020, vol. 440, p. 337-359 discloses treatment options for seizures associated with mutations in KCNA1 and/or KCNQ2, however, CBD is not among the suggested compounds.

Based on these three trials, a botanically derived purified CBD preparation was approved by FDA in June 2018 for the treatment of seizures associated with Dravet and Lennox-Gastaut syndromes.

The applicant has found by way of an open label, expanded-access program that treatment with CBD resulted in a significant reduction in tonic, tonic-clonic, absence and focal seizures with impairment in patients with KCN gene mutation.

## BRIEF SUMMARY OF THE DISCLOSURE

In accordance with a first aspect of the present invention there is provided a cannabidiol (CBD) preparation for use in the treatment of seizures associated with potassium channel (KCN) gene mutation, wherein the KCN gene mutation is in one of *KCNA1* and/or *KCNQ2.*

In a further embodiment, the seizures associated with KCN gene mutation are tonic, tonic-clonic, absence and focal seizures with impairment.

In a further embodiment, the CBD preparation comprises greater than 95% (w/w) CBD and not more than 0.15% (w/w) tetrahydrocannabinol (THC).

Preferably the CBD preparation comprises greater than or equal to 98% (w/w) CBD and less than or equal to 2% (w/w) other cannabinoids, wherein the less than or equal to 2% (w/w) other cannabinoids comprise the cannabinoids tetrahydrocannabinol (THC); cannabidiol-C1 (CBD-C1); cannabidivarin (CBDV); and cannabidiol-C4 (CBD-C4), and wherein the THC is present as a mixture of trans-THC and cis-THC.

Preferably the CBD preparation is used in combination with one or more concomitant anti-epileptic drugs (AED).

Preferably the one or more AED is selected from the group consisting of: clobazam, vigabatrin, phenobarbital, diazepam and zonisamide.

In one embodiment the CBD is isolated from cannabis plant material. Preferably at least a portion of at least one of the cannabinoids present in the CBD preparation is isolated from cannabis plant material.

In a further embodiment the CBD is present as a synthetic preparation. Preferably at least a portion of at least one of the cannabinoids present in the CBD preparation is prepared synthetically.

Preferably the dose of CBD is greater than 5 mg/kg/day. More preferably the dose of CBD is 20 mg/kg/day. More preferably the dose of CBD is 25 mg/kg/day. More preferably the dose of CBD is 50 mg/kg/day.

## DEFINITIONS

Definitions of some of the terms used to describe the invention are detailed below:

Over 100 different cannabinoids have been identified, see for example, Handbook of Cannabis, Roger Pertwee, Chapter 1, pages 3 to 15. These cannabinoids can be split into different groups as follows: Phytocannabinoids; Endocannabinoids and Synthetic cannabinoids (which may be novel cannabinoids or synthetically produced phytocannabinoids or endocannabinoids).

"Phytocannabinoids" are cannabinoids that originate from nature and can be found in the cannabis plant. The phytocannabinoids can be isolated from plants to produce a highly purified extract or can be reproduced synthetically.

"Highly purified cannabinoids" are defined as cannabinoids that have been extracted from the cannabis plant and purified to the extent that other cannabinoids and non-cannabinoid components that are co-extracted with the cannabinoids have been removed, such that the highly purified cannabinoid is greater than or equal to 95% (w/w) pure.

"Synthetic cannabinoids" are compounds that have a cannabinoid or cannabinoid-like structure and are manufactured using chemical means rather than by the plant.

Phytocannabinoids can be obtained as either the neutral (decarboxylated form) or the carboxylic acid form depending on the method used to extract the cannabinoids. For example, it is known that heating the carboxylic acid form will cause most of the carboxylic acid form to decarboxylate into the neutral form.

"Treatment-resistant epilepsy" (TRE) or "intractable epilepsy" is defined as per the ILAE guidance of 2009 as epilepsy that is not adequately controlled by trials of one or more AED.

"Tonic seizures" can be generalised onset, affecting both sides of the brain, or they can be focal onset, starting in just one side of the brain. If a tonic seizure starts in both sides of the brain, all muscles tighten and the subject's body goes stiff. If standing, they may fall to the floor, their neck may extend, eyes open wide and roll upwards, whilst their arms may raise upwards and legs stretch or contract. If a tonic seizure starts in one side of the brain muscles tighten in just one area of the body. Tonic seizures usually last less than one minute.

"Tonic-clonic seizures" consist of two phases: the tonic phase and the clonic phase. In the tonic phase the body becomes entire rigid, and in the clonic phase there is uncontrolled jerking. Tonic-clonic seizures may or may not be preceded by an aura, and are often followed by headache, confusion, and sleep. They may last mere seconds or continue for several minutes. These seizures are also known as a grand mal seizure.

"Absence seizures" also may be called "petit mal" seizures. These types of seizure cause a loss of awareness for a short time. They mainly affect children although can happen at any age. During an absence seizure, a person may: stare blankly into space; look like they're "daydreaming"; flutter their eyes; make slight jerking movements of their body or limbs. The seizures usually only last up to 15 seconds and may occur several times a day.

"Focal Seizures" are defined as seizures which originate within networks limited to only one hemisphere. What happens during the seizure depends on where in the brain the seizure happens and what that part of the brain normally does.

"Focal seizure with impairment" usually start in a small area of the temporal lobe or frontal lobe of the brain and involve other areas of the brain within the same hemisphere that affect alertness and awareness. Most subjects experience automatisms during a focal seizure with impaired consciousness.

## DETAILED DESCRIPTION

## PREPARATION OF HIGHLY PURIFIED CBD EXTRACT

The following describes the production of the highly-purified (>95% w/w) cannabidiol extract which has a known and constant composition.

In summary the drug substance used is a liquid carbon dioxide extract of high-CBD containing chemotypes of *Cannabis sativa* L. which had been further purified by a solvent crystallization method to yield CBD. The crystallisation process specifically removes other cannabinoids and plant components to yield greater than 95% CBD. Although the CBD is highly purified because it is produced from a cannabis plant rather than synthetically there is a small number of other cannabinoids which are co-produced and co-extracted with the CBD. Details of these cannabinoids and the quantities in which they are present in the medication are as described in Table A below.

**Table A: Composition of highly purified CBD extract**

| Cannabinoid | Concentration |
|---|---|
| CBD | > 95% w/w |
| CBDA | NMT 0.15% w/w |
| CBDV | NMT 1.0% w/w |
| $\Delta^9$ THC | NMT 0.15% w/w |
| CBD-C4 | NMT 0.5% w/w |
| > - greater than<br>NMT - not more than | |

**PREPARATION OF BOTANICALLY DERIVED PURIFIED CBD**

The following describes the production of the botanically derived purified CBD which comprises greater than or equal to 98% w/w CBD and less than or equal to other cannabinoids was used in the open label, expanded-access program described in Example 1 below.

In summary the drug substance used in the trials is a liquid carbon dioxide extract of high-CBD containing chemotypes of *Cannabis sativa* L. which had been further purified by a solvent crystallization method to yield CBD. The crystallisation process specifically removes other cannabinoids and plant components to yield greater than 95% CBD w/w, typically greater than 98% w/w.

The *Cannabis sativa* L. plants are grown, harvested, and processed to produce a botanical extract (intermediate) and then purified by crystallization to yield the CBD (botanically derived purified CBD).

The plant starting material is referred to as Botanical Raw Material (BRM); the botanical extract is the intermediate; and the active pharmaceutical ingredient (API) is CBD, the drug substance.

All parts of the process are controlled by specifications. The botanical raw material specification is described in Table B and the CBD API is described in Table C.

**Table B: CBD botanical raw material specification**

| Test | Method | Specification |
|---|---|---|
| Identification:<br>-A<br>-B<br>-C | Visual<br>TLC<br>HPLC/UV | Complies<br>Corresponds to standard (for CBD & CBDA)<br>Positive for CBDA |
| Assay:<br>CBDA + CBD | In-house (HPLC/UV) | NLT 90% of assayed cannabinoids by peak area |
| Loss on Drying | Ph. Eur. | NMT 15% |
| Aflatoxin | UKAS method | NMT 4ppb |
| Microbial:<br>- TVC<br>- Fungi<br>- E.coli | Ph.Eur. | NMT $10^7$ cfu/g<br>NMT $10^5$ cfu/g<br><br>NMT $10^2$ cfu/g |
| Foreign Matter: | Ph.Eur. | NMT 2% |
| Residual Herbicides and Pesticides | Ph.Eur. | Complies |

**Table C: Specification of an exemplary botanically derived purified CBD preparation**

| Test | Test Method | Limits |
|---|---|---|
| Appearance | Visual | Off-white / pale yellow crystals |
| Identification A | HPLC-UV | Retention time of major peak corresponds to certified CBD Reference Standard |
| Identification B | GC-FID/MS | Retention time and mass spectrum of major peak corresponds to certified CBD Reference Standard |
| Identification C | FT-IR | Conforms to reference spectrum for certified CBD Reference Standard |
| Identification D | Melting Point | 65 - 67°C |
| Identification E | Specific Optical Rotation | Conforms with certified CBD Reference Standard; -110° to -140° (in 95% ethanol) |
| Total Purity | Calculation | ≥ 98.0% |
| Chromatographic Purity 1 | HPLC-UV | ≥ 98.0% |
| Chromatographic Purity 2 | GC-FID/MS | ≥ 98.0 % |
| CBDA<br>CBDV<br>THC<br>CBD-C4 | HPLC-UV | NMT 0.15% w/w<br>0.2-1.0% w/w<br>0.01-0.1% w/w<br>0.3-0.5% w/w |
| Residual Solvents:<br>Alkane<br>Ethanol | GC | <br>NMT 0.5% w/w<br>NMT 0.5% w/w |
| Residual Water | Karl Fischer | NMT 1.0% w/w |

The purity of the botanically derived purified CBD preparation was greater than or equal to 98%. The botanically derived purified CBD includes THC and other cannabinoids, e.g., CBDA, CBDV, CBD-C1, and CBD-C4.

In some embodiments, the CBD preparation comprises not more than 0.15% THC based on total amount of cannabinoid in the preparation. In some embodiments, the CBD preparation comprises about 0.01% to about 0.1% THC based on total amount of cannabinoid in the preparation. In some embodiments, the CBD preparation comprises about 0.02% to about 0.05% THC based on total amount of cannabinoid in the preparation.

In some embodiments, the CBD preparation comprises about 0.2% to about 1.0% CBDV based on total amount of cannabinoid in the preparation. In some embodiments, the CBD preparation comprises about 0.2% to about 0.8% CBDV based on total amount of cannabinoid in the preparation.

In some embodiments, the CBD preparation comprises about 0.3% to about 0.5% CBD-C4 based on total amount of cannabinoid in the preparation. In some embodiments, the CBD preparation comprises about 0.3% to about 0.4% CBD-C4 based on total amount of cannabinoid in the preparation.

In some embodiments, the CBD preparation comprises about 0.1% to about 0.15% CBD-C1 based on total amount of cannabinoid in the preparation.

Distinct chemotypes of the *Cannabis sativa* L. plant have been produced to maximize the output of the specific chemical constituents, the cannabinoids. Certain chemovars produce predominantly CBD. Only the (-)-trans isomer of CBD is believed to occur naturally. During purification, the stereochemistry of CBD is not affected.

*Production of CBD botanical drug substance*

An overview of the steps to produce a botanical extract, the intermediate, are as follows:

a) Growing
b) Direct drying
c) Decarboxylation
d) Extraction - using liquid $CO_2$
e) Winterization using ethanol
f) Filtration

g) Evaporation

High CBD chemovars were grown, harvested, dried, baled and stored in a dry room until required. The botanical raw material (BRM) was finely chopped using an Apex mill fitted with a 1 mm screen. The milled BRM was stored in a freezer prior to extraction.

Decarboxylation of CBDA to CBD was carried out using heat. BRM was decarboxylated at 115°C for 60 minutes.

Extraction was performed using liquid $CO_2$ to produce botanical drug substance (BDS), which was then crystalized to produce the test material. The crude CBD BDS was winterized to refine the extract under standard conditions (2 volumes of ethanol at -20°C for approximately 50 hours). The precipitated waxes were removed by filtration and the solvent was removed to yield the BDS.

*Production of botanically derived purified CBD preparation*

The manufacturing steps to produce the botanically derived purified CBD preparation from BDS were as follows:

a) Crystallization using $C_5$-$C_{12}$ straight chain or branched alkane
b) Filtration
c) Vacuum drying

The BDS produced using the methodology above was dispersed in $C_5$-$C_{12}$ straight chain or branched alkane. The mixture was manually agitated to break up any lumps and the sealed container then placed in a freezer for approximately 48 hours. The crystals were isolated via vacuum filtration, washed with aliquots of cold $C_5$-$C_{12}$ straight chain or branched alkane, and dried under a vacuum of <10mb at a temperature of 60°C until dry. The botanically derived purified CBD preparation was stored in a freezer at -20°C in a pharmaceutical grade stainless steel container, with FDA food grade approved silicone seal and clamps.

*Physicochemical properties of the botanically derived purified CBD*

The botanically derived purified CBD used in the clinical trial described in the invention comprises greater than or equal to 98% (w/w) CBD and less than or equal to 2% (w/w) of other cannabinoids. The other cannabinoids present are THC at a concentration of less than or equal to 0.1% (w/w); CBD-C1 at a concentration of less than or equal to 0.15% (w/w); CBDV at a concentration of less than or equal to 0.8% (w/w); and CBD-C4 at a concentration of less than or equal to 0.4% (w/w).

The botanically derived purified CBD used additionally comprises a mixture of both trans-THC and cis-THC. It was found that the ratio of the trans-THC to cis-THC is altered and can be controlled by the processing and purification process, ranging from 3.3:1 (trans-THC:cis-THC) in its unrefined decarboxylated state to 0.8:1 (trans-THC:cis-THC) when highly purified.

Furthermore, the cis-THC found in botanically derived purified CBD is present as a mixture of both the (+)-cis-THC and the (-)-cis-THC isoforms.

Clearly a CBD preparation could be produced synthetically by producing a composition with duplicate components.

Example 1 below describes the use of a botanically derived purified CBD in an open label, expanded-access program to investigate the clinical efficacy and safety of purified pharmaceutical cannabidiol formulation (CBD) in the treatment of seizures associated with KCN gene mutation.

## EXAMPLE 1: CLINICAL EFFICACY AND SAFETY OF PURIFIED PHARMACEUTICAL CANNABIDIOL (CBD) IN THE TREATMENT OF PATIENTS WITH POTASSIUM CHANNEL GENE MUTATION

*Study design*

Subjects were required to be on one or more AEDs at stable doses for a minimum of two weeks prior to baseline and to have stable vagus nerve stimulation (VNS) settings and ketogenic diet ratios for a minimum of four weeks prior to baseline.

Patients were administered botanically derived purified CBD in a 100 mg/mL sesame oilbased solution.

A maximum dose of 50 mg/kg/day could be utilised for patients who were tolerating the medication but had not achieved seizure control; these patients had further weekly titration by 5mg/kg/day.

There were three patients in this study, and each received CBD for various durations of time. Modifications were made to concomitant AEDs as per clinical indication.

Seizure frequency, intensity, and duration were recorded by caregivers in a diary during a baseline period of at least 28 days. Changes in seizure frequency relative to baseline were calculated after at least 2 weeks and at defined timepoints of treatment.

*Statistical Methods:*

Patients may be defined as responders if they had more than 50% reduction in seizure frequency compared to baseline. The percent change in seizure frequency was calculated as follows:

$$\% \text{ change seizure frequency} = \frac{((\text{weekly seizure frequency } \textit{time interval}) - (\text{weekly seizure frequency } \textit{Baseline}))}{(\text{weekly seizure frequency } \textit{Baseline})} \times 100$$

The percent change of seizure frequency may be calculated for any time interval where seizure number has been recorded. For the purpose of this example the percent change of seizure frequency for the end of the treatment period was calculated as follows:

$$\% \text{ reduction seizure frequency} = \frac{((\text{weekly seizure frequency } \textit{Baseline}) - (\text{weekly seizure frequency } \textit{End}))}{(\text{weekly seizure frequency } \textit{Baseline})} \times 100$$

**Results**

*Patient description*

The three patients enrolled in the open label, expanded-access program had mutations in the KCN gene. These patients experienced several different seizure types including tonic, tonic-clonic, absence and focal seizures with impairment and were taking several concomitant AEDs.

The age of patients ranged from 1-10 years, two were male and one was female as detailed in Table 1 below.

**Table 1: Patient demographics, seizure type and concomitant medication**

| Patient Number | Age (years) | Sex | Seizure types | Concomitant AEDs | Etiology |
|---|---|---|---|---|---|
| 1 | 6.67 | M | Tonic-clonic, focal with impairment | ZNS, PHB | *KCNA1* gene mutation |
| 2 | 1.89 | M | Tonic | CLB, VGB | KCNQ2 gene mutation |
| 3 | 10.02 | F | Tonic-clonic, absence | DZP | *KCNQ2* gene mutation |
| CLB = clobazam, VGB = vigabatrin, ZNS = zonisamide, PHB = phenobarbital, DZP = diazepam | | | | | |

*Study medication and concomitant medications*

All three patients were titrated up to at least 25 mg/kg/day of CBD. The average number of concomitant AEDs at the time of starting CBD was two per patient (range: 1-2).

*Clinical changes*

Tables 2A-C illustrate the seizure frequency for each patient as well as the dose of CBD given.

**Table 2A: Seizure frequency data for Patient 1**

| Patient 1 | | | |
|---|---|---|---|
| **Time** | **Seizure Type** | | **Dose CBD (mg/kg/day)** |
| | **Tonic-clonic** | **Focal with impairment** | |
| **Baseline** | 1.0 | 10.0 | - |

(continued)

| | Patient 1 | | |
|---|---|---|---|
| **Time** | **Seizure Type** | | **Dose CBD (mg/kg/day)** |
| | **Tonic-clonic** | **Focal with impairment** | |
| **2 weeks** | 0.0 | 16.0 | 10.0 |
| **4 weeks** | 0.0 | 0.0 | 20.0 |
| **8 weeks** | 1.0 | 1.0 | 25.0 |
| **12 weeks** | 0.0 | 0.0 | 25.0 |
| **16 weeks** | 0.0 | 0.0 | 25.0 |
| **24 weeks** | 0.0 | 5.3 | 25.0 |
| **36 weeks** | 2.0 | 10.0 | 25.0 |
| **48 weeks** | 0.7 | 8.3 | 25.0 |
| **60 weeks** | 0.0 | 0.8 | 25.0 |
| **84 weeks** | 0.0 | 0.0 | 25.0 |

Patient 1 was treated for 84 weeks and experienced a 100% reduction in tonic-clonic seizures and a 100% reduction in focal seizures with impairment over the treatment period.

**Table 2B: Seizure frequency data for Patient 2**

| | Patient 2 | |
|---|---|---|
| **Time** | **Seizure Type** | **Dose CBD (mg/kg/day)** |
| | **Tonic** | |
| **Baseline** | 42.8 | - |
| **2 weeks** | 9.6 | 3.4 |
| **4 weeks** | 32.8 | 10.6 |
| **8 weeks** | 0.0 | 26.0 |
| **12 weeks** | 14.8 | 26.1 |
| **16 weeks** | 6.0 | 26.9 |
| **24 weeks** | 31.0 | 27.7 |

Patient 2 was treated for 24 weeks and experienced a 27.6% reduction in tonic seizures over the treatment period.

**Table 2C: Seizure frequency data for Patient 3**

| | Patient 3 | | |
|---|---|---|---|
| **Time** | **Seizure Type** | | **Dose CBD (mg/kg/day)** |
| | **Tonic-clonic** | **Absence** | |
| **Baseline** | 10.0 | 624.0 | - |
| **8 weeks** | 1.0 | 48.0 | 25.0 |
| **16 weeks** | 1.0 | 200.0 | 25.0 |

Patient 3 was treated for 16 weeks and experienced a 90% reduction in tonic-clonic seizures and a 67.9% reduction in absence seizures over the treatment period.

Overall, patients reported reductions of 27.6-100% in seizures over period of treatment with CBD.

Significantly, one patient (#1) became completely seizure free after 84 weeks of treatment with CBD.

CBD was effective in reducing the frequency of the following seizure types: tonic, tonic-clonic, absence and focal seizures with impairment.

**Conclusions**

These data indicate that CBD was able to significantly reduce the number of seizures associated with KCN gene mutation. Clearly the treatment is of significant benefit in this difficult to treat epilepsy syndrome given the high response rate experienced in all patients.

Of interest is that patients with tonic-clonic seizures (patients 1 and 3) obtained significant benefit.

In conclusion, this study signifies the use of CBD for treatment of seizures associated with KCN gene mutation. Seizure types include tonic, tonic-clonic, absence and focal seizures with impairment for which seizure frequency rates decreased by significant rates, by between 28-100%.

**REFERENCES**

1. Borlot et al. (2020) "KCNTI-related epilepsy: An international multicenter cohort of 27 paediatric cases." Epilepsia, 2020
2. Poisson et al. (2020) "Response to cannabidiol in epilepsy of infancy with migrating focal seizures associated with KCNTI mutations: An open-label, prospective, interventional study", European Journal of Paediatric Neurology, Vol 25; pp 77-81

**Claims**

1. A cannabidiol (CBD) preparation for use in the treatment of seizures associated with potassium channel (KCN) gene mutation, wherein the KCN gene mutation is in one of *KCNA1* and/or *KCNQ2*.

2. A CBD preparation for use according to claim 1, wherein the seizures associated with KCN gene mutation are tonic, tonic-clonic, absence and focal seizures with impairment.

3. A CBD preparation for use according to any of the preceding claims, wherein the CBD preparation comprises greater than 95% (w/w) CBD and not more than 0.15% (w/w) tetrahydrocannabinol (THC).

4. A CBD preparation for use according to any of the preceding claims, wherein the CBD preparation comprises greater than or equal to 98% (w/w) CBD and less than or equal to 2% (w/w) other cannabinoids, wherein the less than or equal to 2% (w/w) other cannabinoids comprise the cannabinoids tetrahydrocannabinol (THC); cannabidiol-C1 (CBD-C1); cannabidivarin (CBDV); and cannabidiol-C4 (CBD-C4), and wherein the THC is present as a mixture of trans-THC and cis-THC.

5. A CBD preparation for use according to any of the preceding claims, wherein the CBD preparation is used in combination with one or more concomitant anti-epileptic drugs (AED).

6. A CBD preparation for use according to claim 5, wherein the one or more AED is selected from the group consisting of: clobazam, vigabatrin, phenobarbital, diazepam and zonisamide.

7. A CBD preparation for use according to any of the preceding claims, wherein the CBD is isolated from cannabis plant material.

8. A CBD preparation for use according to any of the preceding claims, wherein at least a portion of at least one of the cannabinoids present in the CBD preparation is isolated from cannabis plant material.

9. A CBD preparation for use according to claims 1 to 6, wherein the CBD is present as a synthetic preparation.

10. A CBD preparation for use according to claim 9, wherein at least a portion of at least one of the cannabinoids present in the CBD preparation is prepared synthetically.

11. A CBD preparation for use according to any of the preceding claims, wherein the dose of CBD is greater than 5 mg/kg/day.

12. A CBD preparation for use according to any of the preceding claims, wherein the dose of CBD is 20 mg/kg/day.

13. A CBD preparation for use according to any of the preceding claims, wherein the dose of CBD is 25 mg/kg/day.

14. A CBD preparation for use according to any of the preceding claims, wherein the dose of CBD is 50 mg/kg/day.

**Patentansprüche**

1. Cannabidiol(CBD-)Zubereitung zur Verwendung bei der Behandlung von Anfällen im Zusammenhang mit Kaliumkanal(KCN-)Genmutation, wobei die KCN-Genmutation in einem von *KCNA1* und/oder *KCNQ2* ist.

2. CBD-Zubereitung zur Verwendung nach Anspruch 1, wobei die Anfälle im Zusammenhang mit KCN-Genmutation tonische, tonisch-klonische, Abwesenheits- und fokale Anfälle mit Beeinträchtigung sind.

3. CBD-Zubereitung zur Verwendung nach einem der vorhergehenden Ansprüche, wobei die CBD-Zubereitung mehr als 95 % (w/w) CBD und nicht mehr als 0,15 % (w/w) Tetrahydrocannabinol (THC) umfasst.

4. CBD-Zubereitung zur Verwendung nach einem der vorhergehenden Ansprüche, wobei die CBD-Zubereitung mehr als oder gleich 98 % (w/w) CBD und weniger als oder gleich 2 % (w/w) andere Cannabinoide umfasst, wobei die weniger als oder gleich 2 % (w/w) anderen Cannabinoide die Cannabinoide Tetrahydrocannabinol (THC); Cannabidiol-C1 (CBD-C1); Cannabidivarin (CBDV); und Cannabidiol-C4 (CBD-C4) umfassen und wobei das THC als Gemisch von trans-THC und cis-THC vorhanden ist.

5. CBD-Zubereitung zur Verwendung nach einem der vorhergehenden Ansprüche, wobei die CBD-Zubereitung in Kombination mit einem oder mehreren begleitenden antiepileptischen Arzneimitteln (AED) verwendet wird.

6. CBD-Zubereitung zur Verwendung nach Anspruch 5, wobei das eine oder die mehreren AED ausgewählt sind aus der Gruppe bestehend aus: Clobazam, Vigabatrin, Phenobarbital, Diazepam und Zonisamid.

7. CBD-Zubereitung zur Verwendung nach einem der vorhergehenden Ansprüche, wobei das CBD aus Cannabispflanzenmaterial isoliert ist.

8. CBD-Zubereitung zur Verwendung nach einem der vorhergehenden Ansprüche, wobei zumindest ein Teil von zumindest einem der Cannabinoide, die in der CBD-Zubereitung vorhanden sind, aus Cannabispflanzenmaterial isoliert ist.

9. CBD-Zubereitung zur Verwendung nach Anspruch 1 bis 6, wobei das CBD als synthetische Zubereitung vorhanden ist.

10. CBD-Zubereitung zur Verwendung nach Anspruch 9, wobei zumindest ein Teil von zumindest einem der Cannabinoide, die in der CBD-Zubereitung vorhanden sind, synthetisch zubereitet ist.

11. CBD-Zubereitung zur Verwendung nach einem der vorhergehenden Ansprüche, wobei die Dosis von CBD mehr als 5 mg/kg/Tag ist.

12. CBD-Zubereitung zur Verwendung nach einem der vorhergehenden Ansprüche, wobei die Dosis von CBD 20 mg/kg/Tag ist.

13. CBD-Zubereitung zur Verwendung nach einem der vorhergehenden Ansprüche, wobei die Dosis von CBD 25 mg/kg/Tag ist.

14. CBD-Zubereitung zur Verwendung nach einem der vorhergehenden Ansprüche, wobei die Dosis von CBD 50 mg/kg/Tag ist.

**Revendications**

1. Préparation de cannabidiol (CBD) destinée à être utilisée dans le traitement de crises associées à une mutation génique du canal potassique (KCN), dans laquelle la mutation génique du KCN est dans l'un parmi *KCNA1* et/ou *KCNQ2*.

2. Préparation de CBD destinée à être utilisée selon la revendication 1, dans laquelle les crises associées à une mutation génique du KCN sont des crises toniques, tonico-cloniques, d'absence et focales avec altération.

3. Préparation de CBD destinée à être utilisée selon l'une quelconque des revendications précédentes, dans laquelle la préparation de CBD comprend plus de 95 % (p/p) de CBD et pas plus de 0,15 % (p/p) de tétrahydrocannabinol (THC).

4. Préparation de CBD destinée à être utilisée selon l'une quelconque des revendications précédentes, ladite préparation de CBD comprenant une quantité supérieure ou égale à 98 % (p/p) de CBD et inférieure ou égale à 2 % (p/p) d'autres cannabinoïdes, dans laquelle les autres cannabinoïdes représentant une quantité inférieure ou égale à 2 % (p/p) comprennent les cannabinoïdes tétrahydrocannabinol (THC) ; le cannabidiol-C1 (CBD-C1) ; la cannabidivarine (CBDV) ; et le cannabidiol-C4 (CBD-C4), et dans laquelle le THC est présent sous forme d'un mélange de trans-THC et de cis-THC.

5. Préparation de CBD destinée à être utilisée selon l'une quelconque des revendications précédentes, ladite préparation de CBD étant utilisée en combinaison avec un ou plusieurs médicaments anti-épileptiques (AED) concomitants.

6. Préparation de CBD destinée à être utilisée selon la revendication 5, dans laquelle le ou les AED sont choisis dans le groupe constitué par : le clobazam, la vigabatrine, le phénobarbital, le diazépam et le zonisamide.

7. Préparation de CBD destinée à être utilisée selon l'une quelconque des revendications précédentes, dans laquelle le CBD est isolé à partir de matière végétale de cannabis.

8. Préparation de CBD destinée à être utilisée selon l'une quelconque des revendications précédentes, dans laquelle au moins une partie d'au moins l'un des cannabinoïdes présents dans la préparation de CBD est isolée à partir de matière végétale de cannabis.

9. Préparation de CBD destinée à être utilisée selon les revendications 1 à 6, dans laquelle le CBD est présent sous forme d'une préparation synthétique.

10. Préparation de CBD destinée à être utilisée selon la revendication 9, dans laquelle au moins une partie d'au moins l'un des cannabinoïdes présents dans la préparation de CBD est préparée de manière synthétique.

11. Préparation de CBD destinée à être utilisée selon l'une quelconque des revendications précédentes, dans laquelle la dose de CBD est supérieure à 5 mg/kg/jour.

12. Préparation de CBD destinée à être utilisée selon l'une quelconque des revendications précédentes, dans laquelle la dose de CBD est de 20 mg/kg/jour.

13. Préparation de CBD destinée à être utilisée selon l'une quelconque des revendications précédentes, dans laquelle la dose de CBD est de 25 mg/kg/jour.

14. Préparation de CBD destinée à être utilisée selon l'une quelconque des revendications précédentes, dans laquelle la dose de CBD est de 50 mg/kg/jour.

REFERENCES CITED IN THE DESCRIPTION

**Patent documents cited in the description**

- GB 2581517 A
- WO 2019045121 A
- US 2019091174 A
- US 2019247333 A

**Non-patent literature cited in the description**

- **D'ADAMO**. *Neuroscience*, 2020, vol. 440, 337-359
- **ROGER PERTWEE**. Handbook of Cannabis, 3-15
- **BORLOT et al.** KCNTI-related epilepsy: An international multicenter cohort of 27 paediatric cases. *Epilepsia*, 2020, vol. 2020
- **POISSON et al.** Response to cannabidiol in epilepsy of infancy with migrating focal seizures associated with KCNTI mutations: An open-label, prospective, interventional study. *European Journal of Paediatric Neurology*, 2020, vol. 25, 77-81